(19) Europäisches Patentamt
European Patent Office
Office européen des brevets

(11) **EP 2 100 138 B1**

(12) **EUROPEAN PATENT SPECIFICATION**

(45) Date of publication and mention
of the grant of the patent:
**20.06.2012 Bulletin 2012/25**

(21) Application number: **07868154.1**

(22) Date of filing: **27.12.2007**

(51) Int Cl.:
***G01N 27/447*** (2006.01)   ***G01N 21/64*** (2006.01)
***G01N 33/58*** (2006.01)   ***G01N 1/28*** (2006.01)
***G01N 33/53*** (2006.01)

(86) International application number:
**PCT/US2007/026523**

(87) International publication number:
**WO 2008/082670 (10.07.2008 Gazette 2008/28)**

(54) **METHOD FOR INTERNAL STANDARDIZATION OF ASSAYS**

VERFAHREN FÜR INTERNE TESTSTANDARDISIERUNG

PROCEDE DE NORMALISATION INTERNE DE DOSAGES

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **28.12.2006 US 882514 P**

(43) Date of publication of application:
**16.09.2009 Bulletin 2009/38**

(73) Proprietor: **Wako Pure Chemical Industries, Ltd.
Osaka-shi
Osaka 540-8605 (JP)**

(72) Inventors:
• **WADA, Henry G.
Atherton, California 94027 (US)**
• **KAWABATA, Tomohisa
Mountain View, California 94041 (US)**
• **BOUSSE, Luc
Los Altos, California 94022 (US)**

(74) Representative: **von Kreisler Selting Werner
Deichmannhaus am Dom
Bahnhofsvorplatz 1
50667 Köln (DE)**

(56) References cited:
**WO-A2-01/53526       WO-A2-02/052038
US-A- 4 753 888       US-A- 5 605 662
US-A- 5 948 684       US-B1- 6 372 503
US-B1- 6 632 676       US-B2- 6 756 230**

**Description**

<u>Field of the Invention</u>

The invention relates to a method for an internal standardization of assays and a system thereof.

<u>Background of the Invention</u>

An internal standard such as fluorescently labeled DNA can be used as an internal standard to align peaks in electrophoretic assays and also as a quantitative reference marker to normalize the peak signal from fluorescently labeled analyte peaks. The intensity of the analyte peak is the signal used to quantitate the concentration of analyte in tested samples. If there are fluctuations in the assay system that affects the volume of injected sample or the sensitivity of the detection of label, such as fluctuation in exciting laser light intensity, optical focus, etc., the signal for the analyte will be affected to cause error in the measurement of the analyte concentration. By normalizing the signal against an internal standard, it can be controlled for system fluctuations in detection sensitivity, sample volume error, and chip variations such as detection volume variation because of chip channel depth variation.

Normalization methods which rely on the measurement of an optical signal by a known substance used as an internal standard are known e.g. from WO 02/052038. The problem with an internal standard is that it is contained in the sample buffer or other reagent and it would be subject to variation because of, for example, evaporation or condensation that will raise or lower its concentration when stored in a system intended for continuous operation over days, weeks, or even months. Changing the sample buffer every day might be a solution, but it would be inconvenient and expensive for the user. Another possible solution is to have an external calibrator that is run as a sample. This can be used to calibrate the system and adjust the concentration response curve, but it does not control each test for individual chip variation or individual assay variation.

**SUMMARY OF THE INVENTION**

The assay system addresses the problem of using an internal standard for systems such as microfluidic electrophoretic assay systems. With the system, the variation of the internal standard concentration because of factors such as evaporation, condensation or even probe carry-over of probe wash solution can be controlled. The buffer conductivity from when the reagent is fresh can be used by the system to correct the internal standard calibration of analyte signal. The buffer salts are the main contributors to buffer conductivity, and the concentration of these salts by evaporation or dilution by condensation or probe carry-over will change the conductivity of the buffer solution. Therefore, measurement of the buffer conductivity in the chip prior to starting the assay will enable the measurement of the level of evaporation of the reagents used to fill the chip, including the sample buffer. Where it is not possible to directly measure the conductivity of the sample buffer, because of the arrangement of the electrodes in the chip channels, the level of evaporation of one of the reagent components can be measured and the effect of evaporation can be extrapolated to the sample buffer and internal standard.

The same measurement of conductance change can be used as a measure of the chip geometry, because the measured conductance is a function of buffer conductivity, channel length and cross-sectional area. One dominant variable for channel geometry for chips produced by photolithographic processes or molding of thermo-plastic materials may be in the depth dimension. By referencing the conductance to initial measurements of new chips on the instrument to a reference chip on the instrument, it will be possible to normalize assay signals to correct for chip geometry that effects signal intensity, which is predominantly chip channel depth and width, of which, depth may be the predominant variable.

In this way, conductance measurements are a valuable parameter when used in microfluidic electrophoretic assays and can improve the accuracy and reproducibility of these assays.

In accordance with the invention, a normalization method for an assay system is also presented, which includes providing within the assay system a buffer including an internal standard solution; obtaining an electrical conductance measurement of the buffer prior to starting an assay; obtaining a concentration change of the buffer based on the electrical conductance measurement; determining the concentration of the internal standard solution based on the concentration change of the buffer for normalizing assay results.

The buffer of the method is provided for the internal standard and may include a plurality of constituents, one of which is the internal standard solution. The electrical conductivity of the buffer is an aggregate electrical conductivity measurement of the plurality of constituents. The concentration of the internal standard solution may be obtained by extrapolating the concentration change of the buffer. The electrical conductivity of the buffer is approximately proportional to the concentration of salts or ions in the buffer.

A starting concentration of the buffer and a corresponding electrical conductivity measurement of the buffer may be obtained before obtaining the electrical conductivity measurement prior to starting an assay.

The system described in the method may be a microfluidic electrophoretic assay system, in which the assay results may be normalized based on a fluorescent internal standard signal and the concentration of the internal standard solution.

The invention further is a normalization method for an assay system that includes the steps of providing within the assay system a plurality of buffer constituents, one of which includes an internal standard solution; obtaining an electrical conductivity measurement of the plurality of buffer constituents prior to starting an assay; obtaining a concentration change of salts or ions within the plurality of buffer constituents based on the electrical conductivity measurement; and determining a concentration of the internal standard solution based on the concentration change of salts or ions for normalizing assay results.

The invention is further related to a normalization method for an assay system, including providing within the assay system a buffer including an internal standard solution; obtaining an electrical conductance measurement of the buffer prior to starting an assay; and normalizing assay results based on the electrical conductance measurement.

The method may further include the steps of obtaining a concentration change of the buffer based on the electrical conductance measurement; and determining the concentration of the internal standard solution based on the concentration change of the buffer for normalizing the assay results.

The normalization method may further include the steps of comparing the electrical conductance measurement with predetermined data; and normalizing the assay results based on the comparison.

It is to be understood that both the foregoing general description and the following detailed description are exemplary and explanatory only and are not restrictive of the invention, as claimed.

The accompanying drawings, which are incorporated in and constitute a part of this specification, illustrate embodiments of the invention and together with the description, serve to explain the principles of the invention.

## BRIEF DESCRIPTION OF THE DRAWINGS

Figure 1A is an example of a relationship between conductivity and NaCl concentration.
Figure 1 B is an example of a relationship between conductivity and Internal standard concentration.
Figure 2A shows an exemplary graph of data in terms of conductivity v. NaCl concentration.
Figure 2B shows an exemplary graph of data in terms of conductivity v. HyLyte IS concentration.
Figure 3A is an example of a device with wells being connected with microchannels.
Figure 3B is an example of an electrical circuit equivalent to the device shown in Fig. 3A.
Figure 4 is an example of an assay system.
Figure 5A is a graph of an example of normalization using the TB-LB current.
Figure 5B is a graph of an example of normalization using the HO-LB current.

## DESCRIPTION OF THE EMBODIMENTS

Reference will now be made in detail to the present embodiments of the invention, examples of which are illustrated in the accompanying drawings. Wherever possible, the same reference numbers will be used throughout the drawings to refer to the same or like parts.

Uses of fluorescent internal standards for the normalization of the signals measured for fluorescent probes in microfluidic devices may suffer from a lack of stability of the internal standard (IS) signal. If a reagent such as sample dilution buffer or antibody reagent or even a separation buffer includes an internal standard, the concentration of that standard will vary with the extent of evaporation or condensation within the storage container used to store the internal standard. If the container is uncapped during the day to do assays, the extent of evaporation at room temperature can be quite significant, on the order of 10% to 20% in 1 to 2 weeks of use. This will result in bias of assay results that use this internal standard for normalization.

By using the measurement of electrical conductivity or a combination of the measurements of electrical conductivity and fluorescent internal standard signal, the fluorescent signal from microfluidic assays can be normalized such that instrument and chip variations do not introduce large bias in test results. A first measurement, electrical conductivity of the internal standard containing solution, may be used to control for and correct for changes in the internal standard.

Changes in concentration of an internal standard solution because of evaporation or condensation may be correlated with changes in conductivity of the internal standard solution. The IS solution conductivity will be dominated by the concentration of buffer salts in the solution, such as NaCl or other ionic buffers. The conductivity is proportional to the concentration of NaCl so that after determining the initial conductivity, a stable amp meter can be used to measure the extent of change in the NaCl concentration. With the change in the NaCl concentration known, the change in other buffer constituents such as an internal standard may be extrapolated; thereby, a correction in the internal standard concentration can be made during the course of its life time on the microfluidic system, and this IS can be reliably used to normalize assay signals.

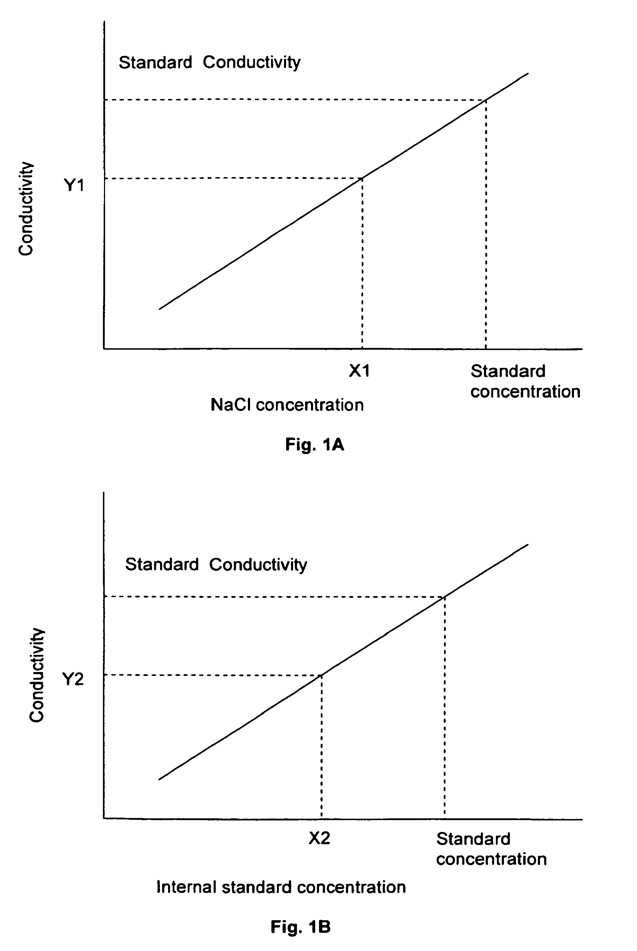

Fig. 1A shows an example of a relationship between conductivity of the solution to the NaCl concentration. Y1 refers to the measured conductivity and X1 refers to the NaCl concentration obtained from the measured conductivity. Fig. 1 B shows an example of a relationship between conductivity of the solution and the concentration of the internal standard. Y2 refers to the calculated conductivity and X2 refers to the internal standard concentration obtained from the calculated conductivity.

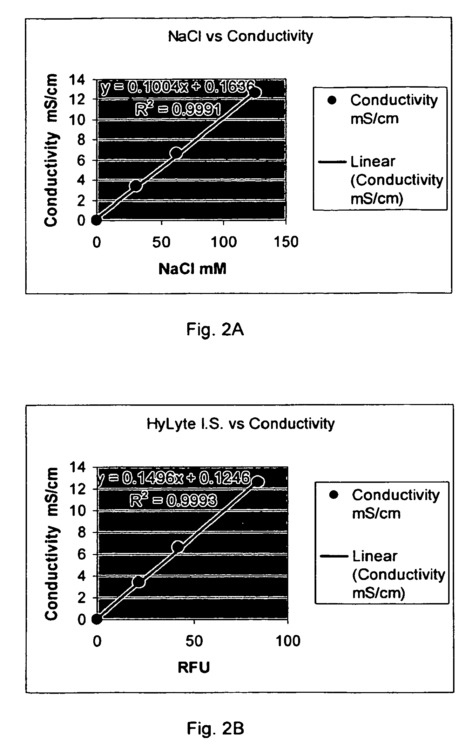

Experimental Measurements using NaCl as buffer salt and HiLyte fluorescent dye as Internal Standard are shown in Figs. 2A and 2B, respectively. There is a correlation of conductivity with buffer salt and IS concentration as measured by relative fluorescent units (RFU). Highest concentration of buffer was 125 mM NaCl and 8.75 uM HiLyte dye that was diluted by water containing 0.05% Tween-20, a non-ionic detergent. The negative control has no NaCl and no HiLyte dye and is deionized water containing 0.05% Tween-20. Measurements were made by a spectrofluorometer and a conductivity electrode. A linear proportionality is demonstrated for conductivity and dye fluorescence at these concentrations of salt which are at relevant assay concentrations.

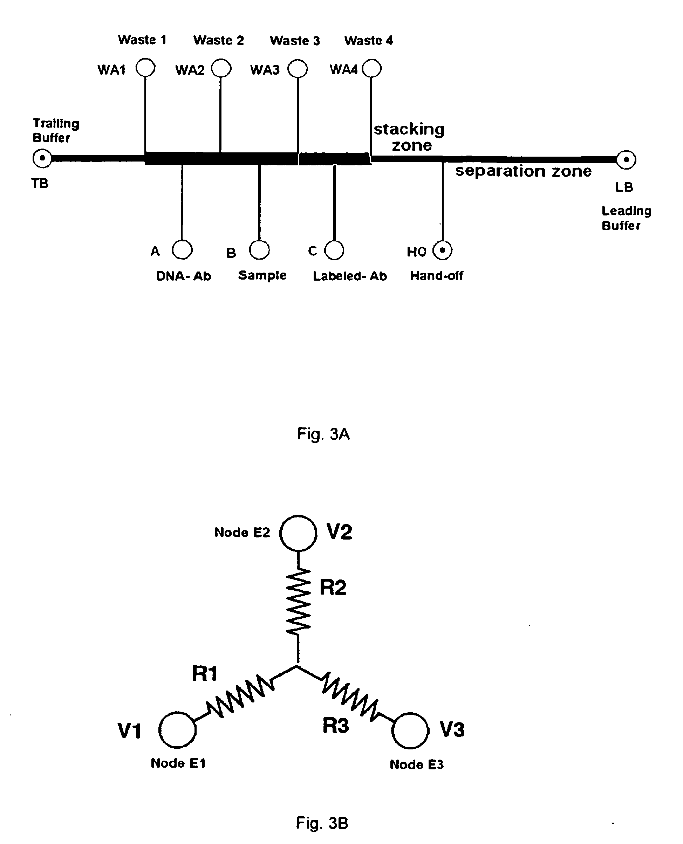

Extrapolation of the IS concentration may be indirect if an aggregate conductivity of several assay reagents is measured. As an example, a microfluidic device as shown in Fig. 3A can be considered. In this figure, all wells are indicated by a circle, but only three wells each marked with a dot contain an electrode. The device has a trailing buffer (TB) well, a leading buffer (LB) well, waste wells (WA) 1 to 4, and A to C Wells with B Well containing an internal standard sample. There is also a hand-off (HO) well disposed between the WA 4 and the LB well.

The electrical equivalent circuit of the microfluidic device shown in Fig. 3B can be represented in terms of a star-shaped network, with three resistors connected to one common internal node. The three wells with the electrodes correspond to the nodes in the electrical equivalent circuit of Fig. 3B. That is, node 1 corresponds to the TB well, node 2 to the HO well, and node 3 to the LB well. By applying a voltage (V2 -V1) between nodes 1 and 2, and measuring the current, the sum of resistances R1 and R2 can be measured:

$$R_{12} = R_1 + R_2 = (V_2\text{-}V_1)/I_{12}$$

The resistances $R_{13}$ and $R_{23}$ between nodes 1 and 3 and nodes 2 and 3, respectively, can be measured in a similar fashion. With the three sums of resistances known, the individual values of the resistances R1 to R3 can be calculated as follows:

$$R_1 = (R_{12} + R_{13} - R_{23})/2$$

$$R_2 = (R_{12} + R_{23} - R_{13})/2$$

$$R_3 = (R_{13} + R_{23} - R_{12})/2$$

With respect to the microfluidic device shown in Fig. 3A, before any electrical tests are run, the device is filled by applying a vacuum simultaneously to the waste wells WA, while the other wells are at atmospheric pressure but filled with a reagent or buffer solution. This creates the filling pattern shown in Fig. 3A. Each channel segment, with one exception, is filled with a solution originating purely from one of the wells. The exception is the segment called the stacking zone, located between the last waste channel intersection and the handoff intersection. That segment will be filled by a mixture of solutions coming from the HO well and from the LB well, in proportion to the hydrodynamic conductivity of these two branches.

In each channel, the geometry, and the origin of the solution that fills each segment are known. In the HO channel (between the HO well and stacking zone) and the LB channel (between the LB well and separation zone), each of these channels is entirely filled with solution coming from the respective well at the end of the channel. Based on the method described above, the electrical resistance of these channels is given by:

$$Rn = \sigma_n^{-1} \frac{Ln}{An} \; ,$$

where Rn is channel resistance

$$G_n = 1/R_n$$

where L is the channel length, A is the channel cross-sectional area, $\sigma$ is the electrical conductivity, R is the electrical resistance, and G is the electrical conductance. Because the geometry of the channel is known, the actual conductivity of the solution coming from the HO and LB wells can be deduced. This measurement therefore is associated with the variations of conductivity that can originate from fluctuations in the making of the buffers, aging during storage, absorption of carbon dioxide from the atmosphere, and evaporation of water while the solution is in the wells.

The situation for the channel connected to the Trailing Buffer (TB) is more complex, because solutions originating from each of the wells are present in that channel. The section of this channel closest to the HO intersection, called the stacking zone will be discussed. During the vacuum loading, the stacking zone is filled with a mixture of HO well solution and LB well solution, in the ratio given by:

$$\alpha_{HO} = \frac{G_{HO}^h}{G_{HO}^h + G_{LB}^h}, \quad \alpha_{LB} = \frac{G_{LB}^h}{G_{HO}^h + G_{LB}^h}$$

where $G_{HO}^h$ is the hydrodynamic conductivity of the HO channel, and $G_{LB}^h$ is the hydrodynamic conductivity of the LB channel. Electrical conductivities are approximately proportional to ionic concentrations in solution, as the data in Figure 2A indicates, and those concentrations will mix according to these filling ratios to get:

$$\sigma_{stacking} = \alpha_{HO}\sigma_{HO} + \alpha_{LB}\sigma_{LB}$$

and with this conductivity, together with the known geometry of the stacking zone, the resistance of the stacking zone is given as follows:

$$R_{stacking} = \sigma_{stacking}^{-1} \frac{L_{stacking}}{A_{stacking}}$$

Subtracting this resistance from the total resistance $R_1$ of the channel connected to the TB well results in the sum of the remaining resistances:

$$R_n = R_1 - R_{stacking} = R_{TB} + R_{DNA-Ab} + R_{sample} + R_{Labeled-Ab}$$

where the resistances on the right hand side correspond to the channel segments filled with the solutions from the TB wells, the DNA-Ab well A, sample well, and Labeled-Ab well respectively.

Due to the lack of more electrical contacts, it is not possible to break down the resistance $R_n$ into further components. However, a goal is to correct for fluctuations due to channel geometry (especially depth), and for evaporation in the wells. Both of these factors are common to the entire chip, including the evaporation which can be assumed to be the same in all the reagent vials and wells, because they have the same geometry, and are exposed to the same external conditions. This can be done by using the electrical conductance $G_n = 1/R_n$ as the normalizing factor.

In the following discussion, it is assumed that $G_n$ is used as the normalizing conductance, since it is the one most influenced by the actual conductance of the sample plug. However, in the assumption that evaporation is similar in all wells, and that thickness variations are also similar throughout the device, any measured conductance could be used. For instance, $G_{23} = 1/R_{23}$ can be used in the terminology established above, which eliminates the need to extract the individual conductance of each arm of the structure. Each particular situation will dictate what the best choice is.

In general, the signal observed by fluorescent optical detection is proportional to the number of fluorescent molecules present in the optical detection volume. Because that number is given by the concentration of fluorescent molecules multiplied by the detection volume, the following equation is obtained:

$$S = f \cdot C \cdot V_{det\,ect}$$

where S is the signal, f is a proportionality constant that takes account of all the optical factors involved, the intensity of the incident light, the efficiency of the fluorescence and so forth, C is the concentration of the fluorescent molecules, and $V_{detect}$ is the detection volume. Since the detection volume $V_{det\,ect}$ is proportional to the channel depth D, this can also be written as:

$$S = f' \cdot C \cdot D$$

With the evaporation of a fraction $\varepsilon$ of the well volume, all concentrations will increase by a factor $1/(1-\varepsilon)$, and this results in:

$$S = \frac{f' \cdot C^0 \cdot D}{1 - \varepsilon}$$

where C° is the concentration of fluorescent molecules before evaporation. The electrical conductance $G_n$ of the channel section described above can vary with channel depth and degree of evaporation. For a channel that has sections of different widths, or filled with different solutions, the following equation in general is given:

$$G_n = \left\{ \sum \sigma_i^{-1} \frac{L_i}{DW_i} \right\}^{-1}$$

However, since the conductivities are proportional to concentrations, which are multiplied by a factor of $1/(1-\varepsilon)$ when evaporation occurs, this can be rewritten as follows in the presence of evaporation, where the electrical conductivities $\sigma_i^0$ are the values before evaporation occurs:

$$G_n = \frac{D}{1 - \varepsilon} \left\{ \sum \left( \sigma_i^0 \right)^{-1} \frac{L_i}{W_i} \right\}^{-1}$$

This equation, together with that of S given above, makes it clear that both quantities vary in the same way as a function of channel depth D and degree of evaporation $\varepsilon$. Therefore, the following equation results:

$$\frac{S}{G_n} = f''C^0$$

a quantity proportional to the concentration of fluorescent molecules before evaporation (by proportionality constant $f''$) and independent of the degree of evaporation, and channel depth. Using this proportionality constant for further analysis, calibration, establishment of standard curves, and so forth, should thus lead to improved accuracy, and independence of externally variable factors such as evaporation or channel depth variations.

A freshly opened calibrator with known analyte concentration (C°) would be measured for the fluorescent signal and $G_n$ to determine $f''$. The $f''$ is then used to calculate the unknown sample analyte concentration (Cˣ) from the measured fluorescence signal and conductivity ratio.

$$\frac{S^x}{G_n} \times \frac{1}{f''} = C^x$$

In summary, if the evaporation increases salt and conductivity by some increment, it is expected that ratios of evaporation would be consistent between each of the containers and wells. That means that any of the possible measurable electrical resistance or conductance values can be used for normalization purposes. That includes conductance values of segments of the devices that do not actually contain the sample.

The method described here may be used for the normalization of microfluidic electrophoretic or other assays that uses electrodes to measure electric current. The current measurement can be used to not only standardize, or normalize an internal standard to correct for evaporation, but also the current can be used to correct for variation in channel dimension, depth and width. The variation of microfluidic devices in geometry can occur primarily by variation in channel depth and width. Micro-fabrication often involves silicon processing techniques that etch glass or silicon masters to form the channels directly or form a mold that is used to produce the channel in thermo-plastic polymers (plastic) substrates. These techniques may introduce subtle variations in the geometry of the channel.

The conductivity of a given microfluidic channel design is a function of the buffer conductivity and the cross-sectional area of the channel. Therefore, there is a very direct relationship between channel depth/geometry and the conductance of the buffer filled channel network. If a nominal cross-section/ conductivity is specified; then, deviation from that nominal value can be calculated by measuring conductance. Furthermore, the assay signal from assays dependent upon channel cross-section such as fluorometric assays can be normalized by measuring channel conductance, using the standardized assay buffers with standard conductivity for freshly opened bottles.

The method describes means to normalize internal standard concentration by measuring an independent parameter, buffer conductance in a system with well characterized reagents and in a well defined microfluidic device. The conductivity of a fresh internal standard solution may be measured whether in isolation or as part of a system that is subject to uniform variation of reagent concentration such as evaporation. Collected conductance measurements may be used to normalize assay signals that are dependent upon microfluidic channel dimensions, such as fluorescence assay signal measurement.

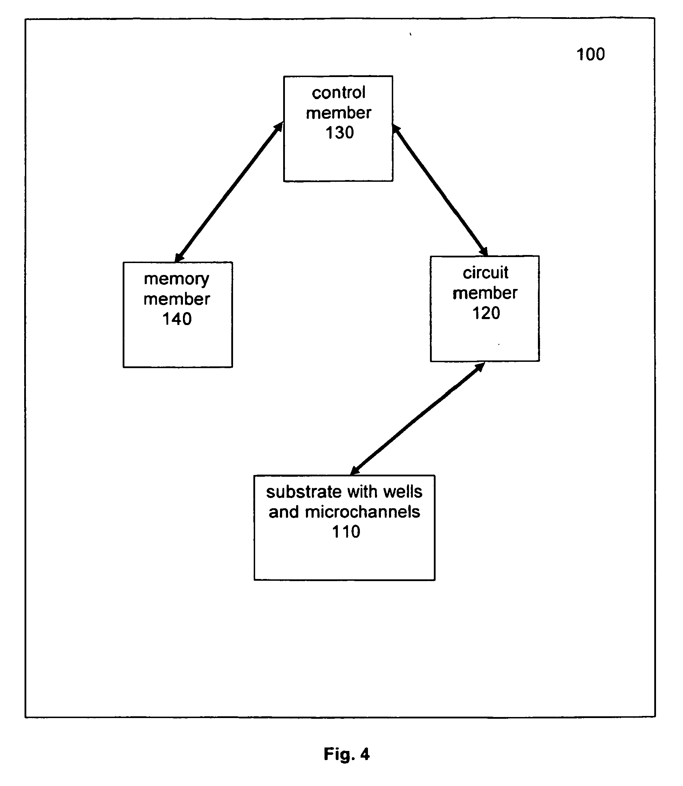

The exemplary method above can be implemented in an assay system 100 as shown schematically in Fig. 4. The exemplary assay system includes a substrate 110 with wells and microchannels connecting the wells, for containing buffer reagents, one of which is for containing an internal standard solution; a circuit member 120 such as a device for measuring electrical conductance between the wells with the buffer reagents; a control member 130 such as a CPU for determining a salt or ion concentration of the buffer reagents from the electrical conductance measurements between the wells; a memory member 140 such as a RAM and hard disk for storing and retrieving predetermined information. The control member 130 extrapolates the salt or ion concentration into a concentration of the internal standard solution based on the predetermined information in the memory member 140.

The assay system 100 may be a microfluidic electrophoretic assay system, and the control member 130 may normalize assay results based on the concentration of the internal standard solution or also with a fluorescent internal standard signal stored in the memory member. The control member 130 may also normalize assay results for geometric dimensional variations of the system by comparing the electrical conductance measurement with standard information stored in the memory member.

The predetermined information may be data for correlating salt or ion concentration information to the concentration of the internal standard solution.

Examples:

Alpha-fetoprotein (AFP) assay was carried out by using a microfluidic device shown in Fig. 3A, according to the description of Example 1 in WO2007/027495. In the examples below, each buffer composition for the assay shown in Table 1 was used as the 100% buffer concentration. Fluorescence end-labeled 2kb DNA was used as an internal standard (IS)

Table 1: Buffer Composition

| Buffer composition for TB well | Buffer composition for DNA-Ab well | Buffer composition for Sample well | Buffer composition for Labled-Ab well | Buffer composition for HO and LB well |
|---|---|---|---|---|
| 75mM Tris | 75mM Tris pH8 | 75mM Tris pH8 | 75mM Tris pH8 | 75mM Tris pH8 |
| 125mM HEPES | 50mM NaCl | 50mM NaCl | 50mM NaCl | 50mM NaCl |
| 0.9% pDMA | 0.9% pDMA | 0.9% pDMA | 0.9% pDMA | 0.9% pDMA |
|  | 200nM 250bp DNA labeled anti-AFP antibody | 100pM AFP-L1 | 100nM Fluorescence lableld second anti-AFP antibody |  |

(continued)

| Buffer composition for TB well | Buffer composition for DNA-Ab well | Buffer composition for Sample well | Buffer composition for Labled-Ab well | Buffer composition for HO and LB well |
|---|---|---|---|---|
| | 250pM Fluorescence labeled 2kbDNA (IS) | | | |

To simulate evaporation and condensation conditions of the buffer composition, the concentrations of buffer components for each well were changed from 80% to 120% in increments of 10%. In an actual assay, the buffer concentration may change by evaporation or condensation but the AFP concentration itself would not change because the analyte sample solution is supplied just before its use. Thus, for this experiment, the AFP antigen sample was mixed with the sample buffer to a final, fixed concentration of 100pM. In each run, referring to Fig.3A, the buffer composition containing the IS was put into the "DNA-Ab" well, the buffer composition containing AFP-L1 into the "Sample" well, the buffer composition containing fluorescence labeled second anti-AFP antibody into the "Labeled-Ab" well, the buffer composition for the TB well into the "TB" well, the buffer composition for the HO and LB wells into the "HO" well and "LB" wells; and by application of vacuum from WA1, WA2, WA3 and WA4 each buffer was introduced into the channel. In each run, capillary electrophoresis (CE) was performed after filling the buffer into the capillary channels.

The antigen-antibody reaction and separation take place in a capillary channel during electrophoresis. Separate IS and antigen-antibody complex signals were detected at a certain point in the capillary channels (separation zone). Current signals between the TB and LB wells and between the HO and LB wells were monitored during electrophoresis, and they were used to normalize the IS peaks.

The IS peak was normalized using the following equation.

$$IS_{Normalized} = IS_{Sample}\left(\frac{A_{reference}}{A_{Sample}}\right)$$

where $IS_{Sample}$ represents the IS peak signal in which condensation or evaporation is assumed to have occurred. $A_{Sample}$ represents the current with condensation or evaporation. $A_{Reference}$ represents the current with no condensation or evaporation. $IS_{Normalized}$ represents the IS signal normalized by the measured current.

The AFP signal was normalized using the following equation.

$$AFP_{Normalized} = AFP_{Sample}\left(\frac{IS_{reference}}{IS_{Normalized}}\right)$$

where $AFP_{Sample}$ represents the AFP peak signal in which condensation or evaporation is assumed to have occured. $IS_{Reference}$ represents the IS signal with no condensation or evaporation. $AFP_{Normalized}$ represents the normalized signal of AFP.

The results of various measured signals and normalization using the TB-LB current and the HO-LB current are given in Table 2 and Table 3 as shown below.

Table 2: TB-LB Current Normalization

| Buffer Concentration | TB-LB current | IS Signal | AFP Signal | AFP normalized by Is_Sample Signal | AFP normalized by IS_Normalized Signal |
|---|---|---|---|---|---|
| 80.0 | 96.0 | 127.1 | 107.5 | 109.7 | 95.7 |
| 90.0 | 109.7 | 134.4 | 115.4 | 111.4 | 108.3 |
| 100.0 | 115.7 | 129.7 | 101.0 | 101.0 | 101.0 |
| 110.0 | 129.1 | 163.7 | 120.8 | 95.7 | 108.7 |

(continued)

| Buffer Concentration | TB-LB current | IS Signal | AFP Signal | AFP normalized by $IS_{Sample}$ Signal | AFP normalized by $IS_{Normalized}$ Signal |
|---|---|---|---|---|---|
| 120.0 | 134.6 | 172.4 | 107.0 | 80.5 | 96.9 |

Table 3: HO-LB Current Normalization

| Buffer Concentration | HO-LB current | IS Signal | AFP Signal | AFP normalized by $IS_{Sample}$ Signal | AFP normalized by $IS_{Normalized}$ Signal |
|---|---|---|---|---|---|
| 80.0 | 82.1 | 127.1 | 107.5 | 109.7 | 91.0 |
| 90.0 | 91.5 | 134.4 | 115.4 | 111.4 | 105.6 |
| 100.0 | 94.1 | 129.7 | 101.0 | 101.0 | 101.0 |
| 110.0 | 106.9 | 163.7 | 120.8 | 95.7 | 106.8 |
| 120.0 | 113.2 | 172.4 | 107.0 | 80.5 | 93.7 |

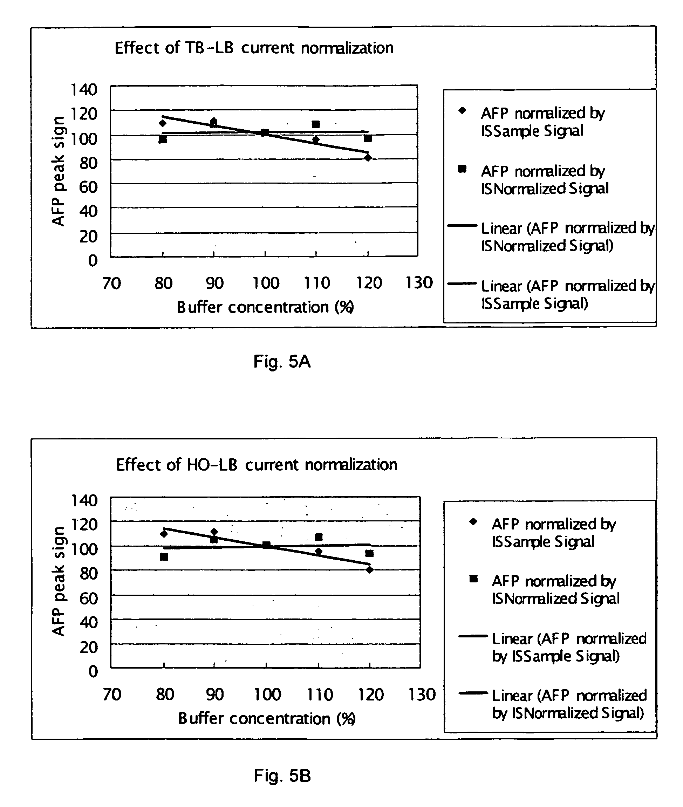

The results of these tables are respectively plotted in Figs. 5A and 5B. In both cases, the AFP signal after normalization by its corresponding $IS_{sample}$ signal decreases with the increasing buffer concentration because the $IS_{sample}$ concentration in the buffer becomes increasingly concentrated. However, for each buffer concentration, the AFP signal after normalization by its corresponding $IS_{Normalized}$ signal gives a value close to the AFP signal at the 100% buffer concentration (no concentration or condensation). That is, the AFP signals normalized by the corresponding normalized IS signals give a flat curve. The example indicates that the IS peak signal can be normalized appropriately even if the buffer concentration in which the IS is incorporated changes by, for example, evaporation or condensation.

Other embodiments of the invention will be apparent to those skilled in the art from consideration of the specification and practice of the invention disclosed herein. It is intended that the specification and examples be considered as exemplary only.

**Claims**

1. A normalization method for an assay system, comprising:

   providing within the assay system a buffer including an internal standard solution;
   obtaining an electrical conductance measurement of the buffer prior to starting an assay;
   obtaining a concentration change of the buffer based on the electrical conductance measurement;
   determining the concentration of the internal standard solution based on the concentration change of the buffer for normalizing assay results.

2. The method according to claim 1, wherein

   (i) the buffer is provided for the internal standard; or
   (ii) the buffer comprises a plurality of constituents, one of which is the internal standard solution; or
   (iii) the electrical conductance of the buffer is approximately proportional to the concentration of salts or ions in the buffer.

3. The method according to claim 1, wherein the buffer comprises a plurality of constituents, one of which includes the internal standard solution; and the electrical conductance measurement of the buffer is an aggregate electrical conductance measurement of the plurality of constituents, preferably the concentration of the internal standard solution is obtained by extrapolating the concentration change of the buffer.

4. The method according to claim 1, further comprising:

   before obtaining the electrical conductance measurement prior to starting an assay, obtaining a starting concentration of the buffer and a corresponding electrical conductance measurement of the buffer.

**5.** The method according to claim 1, wherein the system is a microfluidic electrophoretic assay system.

**6.** The method according to claim 1, wherein the assay results are normalized based on a fluorescent internal standard signal and the concentration of the internal standard solution.

**7.** A normalization method for an assay system, comprising:

providing within the assay system a plurality of buffer constituents, one of which includes an internal standard solution;
obtaining an electrical conductance measurement of the plurality of buffer constituents prior to starting an assay;
obtaining a concentration change of salts or ions within the plurality of buffer constituents based on the electrical conductance measurement;
determining a concentration of the internal standard solution based on the concentration change of salts or ions for normalizing assay results.

**8.** The method according to claim 7, wherein

(i) the electrical conductance measurement of the buffer constituents is an aggregate electrical conductance measurement of the buffer constituents, preferably the concentration of the internal standard solution is obtained by extrapolating the concentration change of salts or ions within the plurality of the buffer constituents; or
(ii) the electrical conductance of the buffer is approximately proportional to the concentration of salt or ions in the buffer.

**9.** The method according to claim 7, further comprising:

before obtaining the electrical conductance measurement prior to starting an assay, predetermining a starting concentration of the buffer constituents and a corresponding electrical conductance measurement of the buffer constituents.

**10.** The method according to claim 7, wherein the system is a microfluidic electrophoretic assay system.

**11.** The method according to claim 7, wherein the assay results are normalized based on a fluorescent internal standard signal and the concentration of the internal standard solution.

**12.** A normalization method for an assay system, comprising
providing within the assay system a buffer including an internal standard solution;
obtaining an electrical conductance measurement of the buffer prior to starting an assay; and
normalizing assay results based on the electrical conductance measurement.

**13.** The normalization method according to claim 12, further comprising:

obtaining a concentration change of the buffer based on the electrical conductance measurement; and
determining the concentration of the internal standard solution based on the concentration change of the buffer for normalizing the assay results.

**14.** The normalization method according to claim 12, further comprising:

comparing the electrical conductance measurement with predetermined data; and
normalizing the assay results based on the comparison.

**Patentansprüche**

**1.** Normierungsverfahren für ein Assaysystem, umfassend:

Bereitstellen eines Puffers, der eine Lösung eines internen Standards umfasst, innerhalb des Assaysystems;
Durchführung einer Messung der elektrischen Leitfähigkeit des Puffers vor Beginn eines Assays;
Erhalten einer Konzentrationsänderung des Puffers anhand der Messung der elektrischen Leitfähigkeit;

Bestimmen der Konzentration der Lösung des internen Standards anhand der Konzentrationsänderung des Puffers zum Normieren der Assayergebnisse.

2. Verfahren gemäß Anspruch 1, wobei

(i) der Puffer als interner Standard bereitgestellt wird; oder

(ii) der Puffer eine Vielzahl von Bestandteilen umfasst, von denen einer die Lösung des internen Standards ist; oder

(iii) die elektrische Leitfähigkeit des Puffers ungefähr proportional zur Konzentration von Salzen oder Ionen in dem Puffer ist.

3. Verfahren gemäß Anspruch 1, wobei der Puffer eine Vielzahl von Bestandteilen umfasst, von denen einer die Lösung des internen Standards umfasst, und die Messung der elektrischen Leitfähigkeit des Puffers eine Messung der elektrischen Gesamtleitfähigkeit der Vielzahl von Bestandteilen ist, wobei vorzugsweise die Konzentration der Lösung des internen Standards durch Extrapolieren der Konzentrationsänderung des Puffers erhalten wird.

4. Verfahren gemäß Anspruch 1, weiterhin umfassend:

bevor man die Messung der elektrischen Leitfähigkeit vor Beginn eines Assays durchführt, Erhalten einer Anfangskonzentration des Puffers und Durchführen einer entsprechenden Messung der elektrischen Leitfähigkeit des Puffers.

5. Verfahren gemäß Anspruch 1, wobei das System ein mikrofluidisches elektrophoretisches Assaysystem ist.

6. Verfahren gemäß Anspruch 1, wobei die Assayergebnisse anhand eines Fluoreszenzsignals eines internen Standards und der Konzentration der Lösung des internen Standards normiert werden.

7. Normierungsverfahren für ein Assaysystem, umfassend:

Bereitstellen einer Vielzahl von Pufferbestandteilen, von denen einer eine Lösung eines internen Standards umfasst, innerhalb des Assaysystems;
Durchführung einer Messung der elektrischen Leitfähigkeit der Vielzahl von Pufferbestandteilen vor Beginn eines Assays;
Erhalten einer Konzentrationsänderung von Salzen oder Ionen innerhalb der Vielzahl von Pufferbestandteilen anhand der Messung der elektrischen Leitfähigkeit;
Bestimmen der Konzentration der Lösung des internen Standards anhand der Konzentrationsänderung der Salze oder Ionen zum Normieren der Assayergebnisse.

8. Verfahren gemäß Anspruch 7, wobei

(i) die Messung der elektrischen Leitfähigkeit der Pufferbestandteile eine Messung der elektrischen Gesamtleitfähigkeit der Pufferbestandteile ist, wobei vorzugsweise die Konzentration der Lösung des internen Standards durch Extrapolieren der Konzentrationsänderung der Salze oder Ionen innerhalb der Vielzahl von Pufferbestandteilen erhalten wird; oder

(ii) die elektrische Leitfähigkeit des Puffers ungefähr proportional zur Konzentration von Salzen oder Ionen in dem Puffer ist.

9. Verfahren gemäß Anspruch 7, weiterhin umfassend:

bevor man die Messung der elektrischen Leitfähigkeit vor Beginn eines Assays durchführt, vorheriges Festlegen einer Ausgangskonzentration der Pufferbestandteile und Durchführen einer entsprechenden Messung der elektrischen Leitfähigkeit der Pufferbestandteile.

10. Verfahren gemäß Anspruch 7, wobei das System ein mikrofluidisches elektrophoretisches Assaysystem ist.

11. Verfahren gemäß Anspruch 7, wobei die Assayergebnisse anhand eines Fluoreszenzsignals eines internen Standards und der Konzentration der Lösung des internen Standards normiert werden.

**12.** Normierungsverfahren für ein Assaysystem, umfassend:

Bereitstellen eines Puffers, der eine Lösung eines internen Standards umfasst, innerhalb des Assaysystems;
Durchführung einer Messung der elektrischen Leitfähigkeit des Puffers vor Beginn eines Assays; und
Normieren der Assayergebnisse anhand der Messung der elektrischen Leitfähigkeit.

**13.** Normierungsverfahren gemäß Anspruch 12, weiterhin umfassend:

Erhalten einer Konzentrationsänderung des Puffers anhand der Messung der elektrischen Leitfähigkeit; und
Bestimmen der Konzentration der Lösung des internen Standards anhand der Konzentrationsänderung des Puffers zum Normieren der Assayergebnisse.

**14.** Normierungsverfahren gemäß Anspruch 12, weiterhin umfassend:

Vergleichen der Messung der elektrischen Leitfähigkeit mit vorher festgelegten Daten; und
Normieren der Assayergebnisse anhand des Vergleichs.

## Revendications

**1.** Procédé de standardisation pour un système d'analyse, comprenant :

la fourniture, à l'intérieur du système d'analyse, d'un tampon comprenant une solution étalon interne ;
l'obtention d'une mesure de conductance électrique du tampon avant de démarrer une analyse ;
l'obtention d'un changement de concentration du tampon sur la base de la mesure de conductance électrique ;
la détermination de la concentration de la solution étalon interne sur la base du changement de concentration du tampon pour standardiser les résultats d'analyse.

**2.** Procédé selon la revendication 1, dans lequel

(i) le tampon est fourni pour l'étalon interne ; ou
(ii) le tampon comprend une pluralité de constituants, dont l'un est la solution étalon interne ; ou
(iii) la conductance électrique du tampon est approximativement proportionnelle à la concentration de sels ou d'ions dans le tampon.

**3.** Procédé selon la revendication 1, dans lequel le tampon comprend une pluralité de constituants, dont l'un comprend la solution étalon interne ; et la mesure de conductance électrique du tampon est une mesure de conductance électrique totale de la pluralité de constituants, de préférence la concentration de la solution étalon interne est obtenue en extrapolant le changement de concentration du tampon.

**4.** Procédé selon la revendication 1, comprenant en outre :

avant l'obtention de la mesure de conductance électrique avant de démarrer une analyse, l'obtention d'une concentration de départ du tampon et d'une mesure de conductance électrique du tampon correspondante.

**5.** Procédé selon la revendication 1, dans lequel le système est un système d'analyse électrophorétique microfluidique.

**6.** Procédé selon la revendication 1, dans lequel les résultats d'analyse sont standardisés sur la base d'un signal étalon interne fluorescent et de la concentration de la solution étalon interne.

**7.** Procédé de standardisation pour un système d'analyse, comprenant :

la fourniture à l'intérieur du système d'analyse d'une pluralité de constituants de tampon, dont l'un comprend une solution étalon interne ;
l'obtention d'une mesure de conductance électrique de la pluralité de constituants de tampon avant de démarrer une analyse ;
l'obtention d'un changement de concentration de sels ou d'ions à l'intérieur de la pluralité de constituants de tampon sur la base de la mesure de conductance électrique ;

la détermination d'une concentration de la solution étalon interne sur la base du changement de concentration de sels ou d'ions pour standardiser les résultats d'analyse.

8.  Procédé selon la revendication 7, dans lequel

    (i) la mesure de conductance électrique des constituants de tampon est une mesure de conductance électrique totale des constituants de tampon, de préférence la concentration de la solution étalon interne est obtenue en extrapolant le changement de concentration de sels ou d'ions à l'intérieur de la pluralité de constituants de tampon ; ou
    (ii) la conductance électrique du tampon est approximativement proportionnelle à la concentration de sels ou d'ions dans le tampon.

9.  Procédé selon la revendication 7, comprenant en outre :

    avant l'obtention de la mesure de conductance électrique avant de démarrer une analyse, la prédétermination d'une concentration de départ des constituants de tampon et d'une mesure de conductance électrique des constituants de tampon correspondante.

10. Procédé selon la revendication 7, dans lequel le système est un système d'analyse électrophorétique microfluidique.

11. Procédé selon la revendication 7, dans lequel les résultats d'analyse sont standardisés sur la base d'un signal étalon interne fluorescent et de la concentration de la solution étalon interne.

12. Procédé de standardisation pour un système d'analyse, comprenant
    la fourniture, à l'intérieur du système d'analyse, d'un tampon comprenant une solution étalon interne ;
    l'obtention d'une mesure de conductance électrique du tampon avant de démarrer une analyse ; et
    la standardisation des résultats d'analyse sur la base de la mesure de conductance électrique.

13. Procédé de standardisation selon la revendication 12, comprenant en outre :

    l'obtention d'un changement de concentration du tampon sur la base de la mesure de conductance électrique ; et
    la détermination de la concentration de la solution étalon interne sur la base du changement de concentration du tampon pour standardiser les résultats d'analyse.

14. Procédé de standardisation selon la revendication 12, comprenant en outre :

    la comparaison de la mesure de conductance électrique à des données prédéterminées ; et
    la standardisation des résultats d'analyse sur la base de la comparaison.

**Standard Conductivity**

Conductivity

Y1

X1 NaCl concentration Standard concentration

**Fig. 1A**

**Standard Conductivity**

Conductivity

Y2

X2 Standard concentration

Internal standard concentration

**Fig. 1B**

## NaCl vs Conductivity

Fig. 2A

## HyLyte I.S. vs Conductivity

Fig. 2B

Fig. 3A

Fig. 3B

100

```
                control
                member
                  130


    memory                      circuit
    member                      member
      140                         120



            substrate with wells
            and microchannels
                  110
```

**Fig. 4**

Fig. 5A

Fig. 5B

**REFERENCES CITED IN THE DESCRIPTION**

**Patent documents cited in the description**

- WO 02052038 A
- WO 2007027495 A